# EUROPEAN PATENT APPLICATION

(11) **EP 4 574 006 A1**
(43) Date of publication of application: **25.06.2025**
(21) Application number: 23220032.9
(22) Date of filing: 22.12.2023
(51) Int. Cl.: A61B 1/00, A61B 1/04, A61B 5/00, G01N 21/64, G02B 21/36

(54) **DATA PROCESSING DEVICE, COMPUTER-IMPLEMENTED METHOD AND MEDICAL OBSERVATION DEVICE**

(71) Applicant: Leica Instruments (Singapore) Pte Ltd, Singapore 608924 (SG)
(72) Inventor: Themelis, George, 608924 Singapore (SG); Pentarakis, Kyriakos, 608924 Singapore (SG)
(74) Representative: Grünecker Patent- und Rechtsanwälte PartG mbB

(57) **Abstract**

Data processing device (170) and computer-implemented method for a medical observation device (100), such as a microscope or endoscope, for observing an object (106) containing at least one fluorophore (116, 118) that emits fluorescence. The data processing device (170) is configured to obtain a first color input pixel (230) of a first digital color input image (130, 114) recorded in a first spectrum (292) and containing a first part (296) of the fluorescence emission signal (222), the fluorescence emission signal being representative of the fluorescence emitted by the at least one fluorophore. The data processing device is configured to obtain a second color input pixel (232) of a second digital color input image (130, 112) recorded in the second spectrum (294) and containing a second part (298) of the fluorescence emission signal, the second spectrum being different from the first spectrum. Further, the data processing device (170) obtains a first set ({S₁}) of signal descriptors (S) representative of the first part of the fluorescence emission signal and obtains a second set ({S₂}) of signal descriptors representative of the second part of the fluorescence emission signal. The data processing device (170) assigns a value (235) to an output pixel (234) in a digital output image (160) depending on the first set of signal descriptors and the second set of signal descriptors, the value being representative of a chemical environment of the at least one fluorescence-emitting fluorophore.

## Description

The invention relates to a data processing device and a computer-implemented method for a medical observation device, such as a microscope or endoscope. The medical observation device is configured to observe an object containing at least one fluorophore that emits fluorescence. The invention further relates to a medical observation device comprising such a digital processing device and to a method for using such a medical observation device, wherein the method comprises the computer-implemented method.

Medical observation devices, such as endoscopes and microscopes are used in surgery and biopsy. The object thus generally may contain biological tissue or materials.

The fluorescence emission of at least one fluorophore is used to provide additional information over the mere reflectance image of the object. The at least one fluorophore may be already present in autofluorescent parts of the object or it may be artificially added to the object. Autofluorescent tissue may be used to highlight the tissue type even without artificial addition of a fluorophore. Other fluorophores are injected into a patient's body or into a cell in order to mark specific regions of interest. If an injected fluorophore stays within the blood stream, it may be used to highlight the location of blood vessels and the direction of flow, i.e. mark anatomical features of the object. Other fluorophores are metabolized in certain chemical or biological environments. Such fluorophores may be used to indicate the functional or metabolic states of regions of the object. As an example, 5-ALA is metabolized intracellularly to PpIX in tumorous cells. Accumulations of fluorescing 5-ALA/PpIX may be used to identify the location and extent of tumors by fluorescence emissions. In general, typical fluorophores that are used to highlight different aspects of biological tissue are ICG, fluorescein and 5-ALA/PplX.

In all these cases, the fluorescence emission of the at least one fluorophore in the object allows data about the object to be obtained, which would not be obtainable from the reflectance image alone, or only be obtainable after extensive image processing.

Thus, an accurate processing of the fluorescence emitted by the object is important for surgery and laboratory applications such as biopsy. The present invention strives to improve this processing in order to provide more accurate data for evaluating the properties of the object.

This objective is solved by a data processing device for a medical observation device, such as a microscope or endoscope, for observing an object containing at least one fluorophore that emits fluorescence, wherein the data processing device is configured: to obtain a first color input pixel of a first digital color input image recorded in a first spectrum and containing a first part of a fluorescence emission signal, the fluorescence emission signal being representative of the fluorescence emitted by the at least one fluorophore; to obtain a second color input pixel of a second digital color input image recorded in a second spectrum and containing a second part of the fluorescence emission signal, the second spectrum being different from the first spectrum; to obtain a first set of signal descriptors representative of the first part of the fluorescence emission signal; to obtain a second set of signal descriptors representative of the second part of the fluorescence emission signal; and to assign a value to an output pixel in a digital output color image depending on the first set of signal descriptors, the value being representative of a state of the at least one fluorescence-emitting fluorophore.

The above objective is also solved by a computer-implemented method for a medical observation device for observing an object containing at least one fluorophore, such as microscope or endoscope, wherein the computer-implemented method comprises the following steps: obtaining a first color input pixel of a first digital color input image recorded in a first spectrum and containing a first part of a fluorescence emission signal, the fluorescence emission signal being representative of the fluorescence emitted by the at least one fluorophore; obtaining a second color input pixel of a second digital color input image recorded in a second spectrum and containing a second part of the fluorescence emission signal, the second spectrum being different from the first spectrum; obtaining a first set of signal descriptors representative of the first part of the fluorescence emission signal; obtaining a second set of signal descriptors representative of the second part of the fluorescence emission signal; and assigning a value to an output pixel in a digital output image depending on the first set of signal descriptors and the second set of signal descriptors, the value being representative of a state of the at least one fluorescence-emitting fluorophore.

The above data processing device and computer-implemented method use two input color images, each recording the fluorescence emission with a different spectrum. Thus more data are available for classifying the fluorescence emission signal compared to just using a single digital color input image. The additional data allow a more accurate distinction between different fluorescence spectra that correspond to different states of the fluorophore.

For example, the fluorescence spectrum of a fluorophore may change depending on its chemical environment. Changes in the fluorescence emission signal are reflected in the set of signal descriptors that represent the fluorescence emission signal or, equivalently, the fluorescence emission spectrum, i.e. the spectrum of the fluorescence emission signal. The set is then mapped to a value, such as an integer value, a float value or a set containing integer and/or float values. The value is thus an indication of the environment of the fluorophore and may be processed further.

As the value is dependent on the fluorescing fluorophore, additional information can be gained over just noting the fluorescence of the at least one fluorophore. The value is indicative of the environmental influence on the fluorophore and thus to the processes at the location of the fluorescing fluorophore.

The digital output image may be any data array where a datum is assigned to a certain location of the object, i.e. a datum corresponding to a pixel. The digital output image does not need to have a standardized image format, such as JPG, GIF or MPG.

The above-identified device and method may be further improved by any one of the following features, which may be arbitrarily combined with one another, each feature having a technical effect of its own. Each of the following features may be used in connection with both the data processing device and the computer-implemented method, independent of whether the particular feature has been described in the context of the data processing device or the computer-implemented method. If, for example, a feature has been described in the context of the computer-implemented method, the data processing device may be likewise configured to execute the feature. If a feature has been described in the context of the data processing device, it may be executed as a step in the computer-implemented method.

Any of the digital input images may contain a plurality of input pixels. Each input pixel represents the light received from a specific location on the object. Each input pixel corresponds to a specific location on the object. If the input image data contain more than one digital input image, a specific location on the object may be represented by more than one input pixel. Input pixels that represent the same location on the object are termed "corresponding pixels" as is usual in the art.

An input pixel may be a color pixel or a monochrome pixel. A color input pixel comprises color space coordinates that define the color of the input pixel in a color space. The color space coordinates represent at least some of the color appearance parameters, which include hue, lightness, brightness, chroma, colorfulness and saturation. If a tristimulus color space such as RGB is used, each input pixel comprises three color space coordinates R, G, B. The color space coordinate R defines the intensity of the red color band, the color space coordinate G defines the intensity of the green color band, and the color space coordinate B defines the intensity of the blue color band. Other color spaces, such as HSV, CIELAB or CIELUV, use other color space coordinates. A monochrome input pixel just indicates light intensity in the spectral band where the light is recorded. It thus has only a single color space coordinate.

Throughout this text, if a digital input image is a color image, it is termed a "digital color input image". A multispectral or hyperspectral image is considered a color image. If a digital input image is a monochrome image, it is termed "digital monochrome input image". If, in a specific context, it does not matter whether the digital input image is a color or a monochrome image, the generic term "digital input image" is used. A "digital input image" may thus be a "digital color input image" or a "digital monochrome input image". The same terminology is used for the digital output image.

Any of the digital input images may contain one or more signals, such as the fluorescence emission signal or part thereof. A "signal" in this context represents a recorded quantity that is generated by a physical phenomenon. The fluorescence emission signal is thus a record of the light generated by the fluorescence emission and recorded as a video or image signal. A reflectance signal, in contrast, is a recorded video or image signal that is generated by the reflection of light off a body.

The first and second spectrum are preferably non-overlapping. An overlap which is solely due to unavoidable filter leakage may be allowed. Preferably, the first and the second spectrum are complementary. In particular, the first and the second spectrum may complement each other to continuously cover the entire visible range of the spectrum. In a further improvement, at least one of the first and the second spectrum may include a passband which covers or is contained in the NIR region. The visible light range comprises or consists of light having wavelengths longer than about 380 nm and shorter than about 750 nm. The NIR range of light extends from about 750 nm to about 1400 nm or to about 2500 nm.

As stated above, the value assigned to an output pixel should be representative of the chemical environment at the location of the object, which is imaged onto the output pixel and in which the at least one fluorescence-emitting fluorophore is located. The chemical environment may alter the fluorescence emission spectrum of the fluorophore and thus change the set of signal descriptors. The chemical environment may be represented or described by a chemical parameter which e.g. corresponds to the concentration of a specific ion or macromolecule. The set of signal descriptors and the value are, via the dependency of the fluorescence emission spectrum on the chemical parameter, representative of the chemical parameter. An example of such a chemical parameter is the pH level.

The change in the fluorescence emission spectrum depending on the chemical parameter may manifest itself in different aspects of the shape of the spectrum. In one instance, the number and/or wavelengths and/or relative intensities of one or more peaks of the fluorescence emission spectrum may change depending on the chemical parameter. Alternatively or accumulatively, the energy contained in the off-peak areas of the fluorescence emission spectrum may alter depending on the chemical parameter. Alternatively or cumulatively, the width of the one or more peaks of the fluorescence emission spectrum may depend on the chemical parameter.

Also, the shape of secondary peaks in the fluorescence emission spectrum may change depending on the chemical parameter. As an example, 5-ALA/PpIX exhibits a pH-dependent fluorescence spectrum, where the shape, e.g. the wavelength of the peak emission frequency depends on the acidity of the environment.

Different types and/or gradings of tumors have different chemical environments, which cause any of the above-described changes to the fluorescence emission spectrum of a fluorophore, such as 5-ALA/PplX, in the tumor. The different spectra are represented by different sets of signal descriptors and thus by different values assigned to the output pixel onto which the tumor is imaged.

Specifically, different grades of tumors have different acidity. Thus, the acidity-depending fluorescence emission spectrum of 5-ALA/PpIX may be used to determine the grading of tumorous tissue.

The data processing device may be configured to map the set of signal descriptors to a predefined range of values, e.g. using linear mapping. The predefined range of values may correspond to an upper and a lower bound of the chemical parameter. For example, the upper bound may correspond to a maximum pH level and the lower bound may correspond to a minimum pH level. The upper and lower bounds are each represented by a different set of signal descriptors. The set of signal descriptors that is representative of (a fluorescence emission spectrum at) the upper bound is mapped to a first value and the set of signal descriptors that is representative of (a fluorescence emission spectrum at) the lower bound is mapped to a second value. A set of signal descriptors that is representative of (a fluorescence emission spectrum at) a chemical parameter which is located between the upper and the lower bound is mapped onto a value which is located between the first and the second value.

In one embodiment, the value assigned to the output pixel may be a color. The value may correspond, in particular, to at least one color appearance parameter. In this case, the state of the at least one fluorescing fluorophore or the chemical environment is color-coded. The color may be represented in color space coordinates of the color space, in which the digital output image is represented.

Using a color as the value is a quick way to code the state of the fluorophore and present it in an output image. If the value is not represented as a color in a color space, it may be contained in the metadata of the digital output image or the respective output pixel.

In a very simple embodiment, the first color input pixel may comprise first color space coordinates and the second color output pixel may comprise second color space coordinates, the first set of signal descriptors may comprise or correspond to the first color space coordinates and the second set of signal descriptors may comprise or correspond to the second color space coordinates. Thus, the signal descriptors may simply correspond to the colors recorded at the first and second input pixel. This is accurate if there are no or only negligible additional signals other than the fluorescence emission signal recorded at the first and second input pixel or by the first and second digital input image.

To extract the fluorescence emission signal from the first and/or the second digital input image, spectral unmixing may be used. The set of signal descriptors may be obtained directly as a result of the spectral unmixing. The data processing device may be configured to apply spectral unmixing to the first color input pixel to obtain the first set of signal descriptors; and to apply spectrum unmixing to the second color input pixel to obtain the second set of signal descriptors. The data processing device may comprise a spectral unmixing routine.

In spectral unmixing, it is assumed that the color of an input pixel results from the sum of a plurality of known spectra. The known spectra are termed "endmembers". The color of an input pixel is computed as a sum of the endmembers. The contributions or, equivalently, coefficients of the endmembers that result in the color of the input pixel may be used as the set of signal descriptors. As an endmember, the (known or assumed) fluorescence emission spectra of the at least one fluorophore in predetermined chemical environments or at predetermined chemical parameters, such as the pH level, may be used.

For example, one endmember may represent the fluorescence emission spectrum of 5-ALA/PpIX in an acidic environment at a predetermined pH level, such as a pH level of about 4 or about 5 or less. Another endmember may represent the fluorescence emission spectrum of 5-ALA/PpIX in an alkaline environment at a predetermined pH level, such as a pH level of about 8 or about 9 or more.

Alternatively, the respective passbands of the first and second spectrum may be used as endmembers. For example, the first spectrum may comprise at least one, preferably at least two passbands. The second spectrum may comprise at least one passband, which may be located between two passbands of the first spectrum. Another passband of the second spectrum may be located in the NIR range. At least one passband of the second spectrum overlaps, contains or is contained within the fluorescence emission spectrum of the at least one fluorophore, for example 5-ALA/PplX. This passband may, for example, comprise at least one peak emission frequency of the fluorescence emission spectrum of the at least one fluorophore.

The data processing device may be configured to assign a color to the color output pixel by e.g. applying a mapping routine to a union set of the first set and the second set of signal descriptors. The union set of the first set and the second set corresponds to a tuple formed by all the signal descriptors of the fluorescence emission signal.

The mapping routine may be a linear transformation function and in particular comprise a color conversion matrix. For example, the union set of the first and second set of signal descriptors may be treated as a vector (or matrix) which is multiplied with the color conversion matrix. The color coordinates of the output pixel may be obtained directly from this multiplication.

The mapping routine and/or the color conversion matrix may be stored in a memory of the data processing device. The mapping routine and/or the color conversion matrix may be determined experimentally in a calibration process. In this calibration process, at least some sets of signal descriptors are computed for known fluorescence emission spectra. The known fluorescence emission spectra preferably correspond to known chemical parameters. The mapping routine and/or the color conversion matrix may then be determined by assigning specific colors to these known sets of signal descriptors in the calibration process.

In one particular embodiment, the data processing device may be configured to map the first and second set of signal descriptors, or the union set, to a first value, if the first and second sets, or the union set, are representative of the fluorescence emission spectrum of the at least one fluorophore in a first chemical environment. The first chemical environment is preferably represented by a first figure of a chemical parameter such as the pH level. Further, the data processing device may be configured to map the first and second set of signal descriptors, or the union set, to a second value, if the first and second sets, or the union set, are representative of the fluorescence emission spectrum of the at least one fluorophore in a second chemical environment. The second chemical environment is preferably represented by a second figure of the chemical parameter such as the pH level. The data processing device may further be configured to map the first and second set of signal descriptors, or the union set, to a third value or color if the first and second signal descriptors, or the union set, are representative of a third chemical environment. The third chemical environment is preferably represented by a third figure of the chemical parameter that lies between the first and the second figure. For this mapping, the mapping routine may be used. The position of the third value relative to the first and/or second value or color may correspond to position of the third figure relative to the first and/or second figure.

If the value corresponds to color, the first value may correspond to a first, predetermined color and the second value may correspond to a second, predetermined color. Each color may be defined by at least one color appearance parameter depending on the sets of signal descriptors. When a color is assigned to the output pixel, any color appearance parameter may be assigned to the output pixel depending on the set of signal descriptors.

All third colors may be mapped onto a curve which connects the first and the second color in a color space.

The chemical parameter, e.g. in case of 5-ALA/PpIX the pH level, may be indicative of the tumor grade. For example, an acidic pH may be indicative of a high-grade tumor, e.g. a high-grade glioma, and an alkaline pH of a low-grade tumor, e.g. a low-grade glioma. If the first value or color represents a highest tumor grade and the second value or color represents a lowest tumor grade, the third value or color may be indicative of the tumor or glioma grading between the highest and lowest tumor grade. The larger the difference of the third value or color from the first value or color, the lower the tumor or glioma grade.

In one embodiment, the sets of the first and second signal descriptors, or the union set, are mapped to the first value or color if the first and second set of signal descriptors, or the union set, is representative of a fluorescence emission spectrum having a peak at a first wavelength. In case of 5-ALA/PpIX the first wavelength may be around 634 nm. The first and second set of signal descriptors, or the union set, may be mapped to the second value or color, if the first and second set of signal descriptors, or the union set, are representative of a fluorescence emission spectrum having a peak at a second wavelength. In case of 5-ALA/PpIX the second wavelength may be around 620 nm. The third value or color may be representative of a first and second set of signal descriptors, or of a union set, that is representative of a fluorescence emission spectrum having at least one peak between 620 and 634 nm.

According to one aspect, the color that is assigned to the output pixel may be a pseudocolor or a false color. In the case of a pseudocolor, a different hue may be assigned to the output pixel depending on the set of signal descriptors. In the case of a false color, the same hue, but at a different intensity may be assigned to the output pixel depending on the set of signal descriptors.

The medical observation device that includes the digital processing device in any of the above-described embodiments may further comprise a first digital color camera for recording the first digital color input image of the object in the first spectrum; and a second digital color camera for recording the second digital color input image of the object in the second spectrum.

The digital processing device may, in one instance, be an embedded system of the medical observation device. In another instance, the digital processing device may be part of a computer which provides an interface to the medical observation device.

The first digital color camera may be a white-light color camera, in particular having an observation filter system adapted to record a white-light reflectance image of the object. The second digital color camera may be a fluorescence-light color camera, in particular having a fluorescence filter system adapted to record the fluorescence emission spectrum of the at least one fluorophore. The fluorescence light color camera may be adapted to record the majority of wavelengths contained in the fluorescence emission spectrum of the at least one fluorophore. The second spectrum may be more narrow-band compared to the first spectrum, i.e. contain less wavelengths than the first spectrum.

The medical observation device may further comprise an observation filter set located in front of the first digital color camera and comprising an observation stopband; a fluorescence filter set located in front of the second digital color camera and comprising a fluorescence passband; wherein the fluorescence passband is located in the observation stopband. In a particular instance, the fluorescence passband may extend from a wavelength of about 610 nm to a wavelength of about 650 nm. In particular, the wavelength 620 nm and 634 nm may be contained in the fluorescence passband.

The observation passband may contain the wavelength from about 650 nm to about 610 nm. A second observation passband may contain wavelengths shorter than about 610 nm.

The observation stopband and the fluorescence passband may be non-overlapping. More particularly, the observation stopband may correspond to the fluorescence passband. The fluorescence passband may be located between two observation passbands as described above.

The observation and/or fluorescence filter set are preferably located in the optical path of the medical observation device leading to the first and/or second digital color camera, respectively.

A method for using a medical observation device for observing an object containing at least one fluorescing fluorophore may comprise the steps of recording a first digital color input image in a first spectrum, the first digital color input image comprising a plurality of first color input pixels; recording a second digital color input image in a second spectrum, the second digital color input image comprising a plurality of second color input pixels, the first spectrum being different from the second spectrum; and carrying out the computer-implemented method in any of the embodiments described above.

The initially mentioned objective is also achieved by a method for using a medical observation device, such as an endoscope or microscope, wherein the method comprises the steps of recording the input image data and carrying out the computer implemented method in any of the above-described configurations.

Finally, the invention is also concerned with a computer program product and/or a computer-readable medium comprising instructions, which when executed by a computer causes the computer to carry out the computer implemented method in any of the above configurations.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

In the following, the invention is described exemplarily with reference to various examples and with reference to the drawings. The combination of features that are described and/or shown in the drawings and/or examples is not to be considered as limiting. For example, features may be omitted from an embodiment if it has a technical effect e.g. as explained above, that is not needed in a specific application. Conversely, a feature described above that is not part of an embodiment described below may be added if the technical effect associated with this particular feature is beneficial in a specific application.

Throughout the description and the drawings, the same reference numerals are used for elements that correspond to each other with respect to function and/or structure.

In the drawings,
- Fig. 1: shows a schematic representation of a medical observation device;
- Fig. 2: shows a schematic representation of the generation of a digital output image from two digital color input images containing a fluorescence emission signal;
- Fig. 3: shows a schematic representation of the dependency of fluorescence emission spectra on the chemical environment of the fluorescing fluorophore and the assignment of a color depending on the fluorescence emission spectrum;
- Fig. 4: shows a schematic representation of how signal descriptors are obtained and multiplied with a color conversion matrix;
- Fig. 5: shows a schematic representation of the method for obtaining a digital output image;
- Fig. 6: shows a schematic representation of a generic medical observation device.

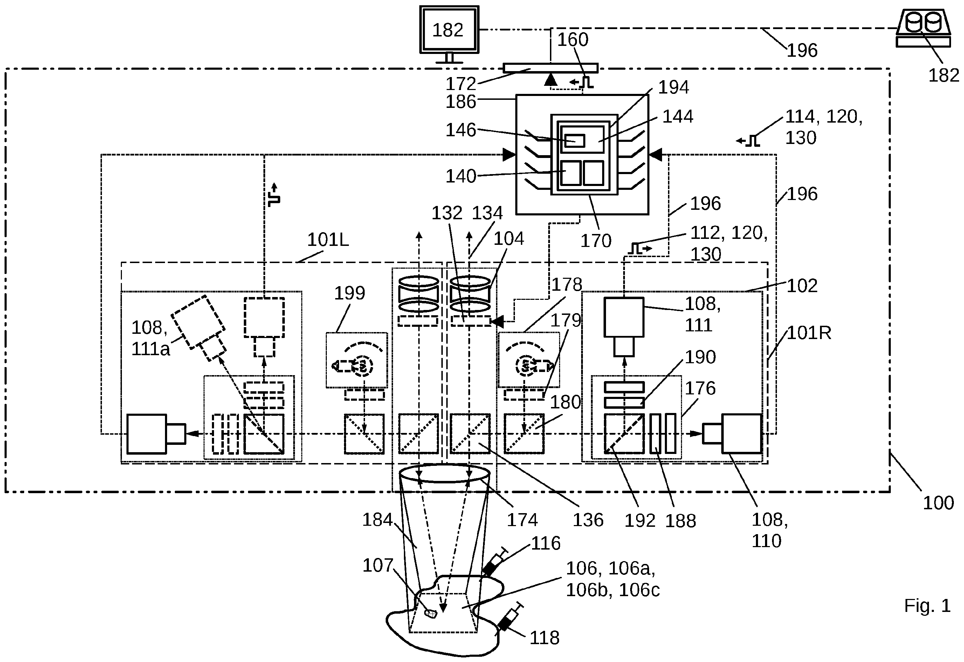

Fig. 1 shows schematically a medical observation device 100. The medical observation device 100 may be a microscope or an endoscope, the difference between a microscope and an endoscope being primarily that, in an endoscope (not shown), an object 106 is viewed through optical fibers that are brought into vicinity of the object 106 to be investigated, e.g. by insertion into a body containing the object, whereas, in a microscope, an objective 174 is directed onto the object. Although the medical observation device of Fig. 1 is a microscope, the following description also applies to an endoscope.

The medical observation device 100 may be a medical observation device used in surgery. The medical observation device 100 may also be a medical observation device used in a laboratory, such as a laboratory microscope. The object 106 to be investigated may consist of or comprise biological tissue 107. The object 106 may be a part of a patient's body that is located within the field of view of the medical observation device 100.

The object 106 may contain one or more fluorophores 116, 118. At least one fluorophore 116 may be a fluorophore that is naturally contained in the object. For example, bone and blood naturally contain fluorophores. At least one fluorophore 118 may be artificially added to the object 106, e.g. by injecting it into the biological tissue 107. Examples of fluorophores 118 that may be artificially added to the object 106 are ICG, fluorescein and/or 5-ALA.

The medical observation device 100 as shown may be a fluorescence-imaging device. Thus, the medical observation device may be configured to view and preferably excite the fluorescence of the one or more fluorophores 116, 118.

The medical observation device 100 may be a stereoscopic device as is exemplarily shown in Fig. 1. It may thus comprise two identical subassemblies 101L and 101R for each of the two stereoscopic channels. As the two subassemblies 101L, 101R are identical with respect to function and structure, the following description focuses on the right subassembly 101R, but applies identically to the left stereoscopic channel 101L.

The medical observation device 100 may alternatively be a monoscopic device. In this case, only one of the two subassemblies 101L, 101R may be present. For a monoscopic medical observation device 100, the following description therefore applies as well.

The medical observation device 100 in operation provides input image data 120. The input image data 120 are representative of an imaged scene, i.e. the part of the object that is within the field of view 184 of the medical observation device 100.

The input image data 120 may comprise one or more different digital input images 130. The digital input images may be digital color input images 130, digital monochrome input images 130 or a combination of at least one digital color input image 130 and at least one digital monochrome input image 130.

If the input image data 120 contain a plurality of digital input images 130, the different digital input images 130 should contain different spectral information. In such a case, each digital input image 130 of the input image data may be recorded at different wavelengths, preferably with no spectral overlap, where unavoidable filter leakage is not considered as overlap. Preferably, the imaged spectra, in which the different digital input images 130 of the input image data 120 are recorded, are non-overlapping. According to another aspect, the imaged spectra are complementary. In this case, the stopbands and passbands of the imaged spectra complete each other to form seamlessly or at least almost seamlessly a continuous input spectrum, which is split into the imaged spectra.

For generating the input image data 120, the digital imaging system 102 may comprise one or more digital cameras 108, which are preferably digital color cameras. The number of digital input images 130 contained in the input image data 120 may depend on, in particular be equal to the number of cameras 108 used for generating the input image data 120. Depending on type and setting of digital camera 108, a digital input image 130 may be a color image or a monochrome image.

The medical observation device 100 may be configured to record in the input image data 120 the (auto)fluorescence of the fluorophore 116 that occurs naturally in the object, and the fluorescence of at least one fluorophore 116, 118 that has been added artificially. For example, the one or more fluorophores 116, 118 may have been injected into a patient's body to mark specific areas of interest, such as tumors. For recording a fluorescing fluorophore, at least one of the digital cameras 108, a digital fluorescence-light camera 111, has an imaged spectrum that comprises or is contained in the (known) fluorescence emission spectrum of the fluorophore of which fluorescence is to be recorded.

The medical observation device 100 may also be configured to record the light reflected off the object in the input image data 120. This may be done simultaneously or sequentially to recording the fluorescence emission of the fluorophore.

To record the reflectance of the object, another digital camera may be used other than the one used for recording the at least one fluorescing fluorophore 116, 118. This digital camera 108, a digital reflectance camera 110, may be used to provide a preferably white-light reflectance image of the object 106 in particular in the wavelengths excluding the wavelengths recorded by the other digital camera 108, i.e. the fluorescence emission spectrum or part thereof.

According to one embodiment, however, it is preferred that the medical observation device 100 is configured to use both the digital camera that is used for recording the fluorescence emission of the at least one fluorophore, and the digital camera that is used for recording the reflectance image, to record a fluorescence image of the object. The fluorescence image recorded by the two or more cameras 108 has additional spectral information over a reflectance image recorded by just one camera 108.

Each of the two or more cameras 108 records a separate digital input color image 130. The medical observation device 100 may be configured to generate a digital multispectral reflectance input image from the plurality of digital input images 130, which are preferably registered with respect to one another.

In one embodiment, the digital imaging system 102 may comprise digital cameras 108, a digital reflectance camera 110, and one or more digital fluorescence-light cameras 111, 111a. A second (or third) digital fluorescence-light camera 111a is optional. Each fluorescence-light camera should record light in a different, preferably non-overlapping imaged spectrum that is preferably complementary to all other imaged spectra.

The camera 110 is configured to record a first digital input color image 114 containing first input pixels and at least one of the cameras 111, 111a is configured to record a second digital input color image 112 containing second input pixels. The first and the second digital color input images 112, 114 are preferably registered relative to one another. The first and second input pixels are preferably corresponding pixels.

In Fig. 1, the second digital fluorescence-light camera 111a is only shown in the left stereoscopic channel 101L, but of course may also be present in the right stereoscopic channel 101R. Alternatively, the digital fluorescence-light camera of one stereoscopic channel may be used as the (first) digital fluorescence-light color camera 111 and the digital fluorescence-light camera of the other stereoscopic channel may be used as the second fluorescence-light camera 111a. The cameras 110, 111, 111a may each be a color camera or a monochrome camera. A multispectral camera or a hyperspectral camera is considered a color camera.

The digital reflectance camera 110 is configured to record a digital reflectance input image 114 as the first digital input image. The digital reflectance input image 114 is representative of the reflectance of the object 106. The digital reflectance camera 110 is preferably configured to record a digital input image 130 in a wide spectral range within the visible light spectrum. Thus, the digital input image 130 recorded by the digital reflectance camera represents closely the natural colors of the object 106. This is important if the digital reflectance camera 110 is used to provide the user with an image of the object which comes as close as possible to the human perception of the object. The digital reflectance camera 110 may be a CCD, CMOS or multispectral or hyperspectral camera. The digital reflectance camera 110 may also be used to record the fluorescence emission of a fluorophore.

Each of the at least one digital fluorescence-light camera 111, 111a is configured to record a different digital fluorescence-light input image, i.e. a digital input image 130, which is recorded in the fluorescence spectrum or the fluorescence spectra of the at least one fluorophore 116, 118. Each fluorescence-light camera 111, 111a may be configured to record the fluorescence of a different fluorophore. Each digital fluorescence-light image 112 or the combination of digital fluorescence-light input images may correspond to the second digital input image 112.

The fluorescence-light camera 111 may be configured to record the digital fluorescence-light image only in one or more narrow bands of light. These narrow bands should overlap the fluorescence spectrum or spectra of the one or more fluorophores 116, 118 of which fluorescence is to be recorded. Preferably, the fluorescence spectra of the different fluorophores 116, 118 are at least partly separate, preferably completely separate, i.e. non-overlapping, so that the fluorescence-light camera 111 may record a digital color input image 130 representing two separate fluorescence bands that are spaced from one another.

Alternatively, if two fluorescence-light cameras 111, 111a are provided, each fluorescence-light camera 111, 111a preferably captures the fluorescence emission of a different fluorophore.

The at least one fluorescence-light camera 111, 111a may be a monochrome camera, a CCD, CMOS or multispectral or hyperspectral camera. Preferably, the white-light color camera 110 and the at least one fluorescence-light color camera 111 are of the same type, although this is not necessary.

As stated above, the digital reflectance camera 110 and the at least one fluorescence-light camera 111, 111a may be used to record a reflectance input image of the object 106.

Any combination of the cameras 110, 111 and 111a may be combined into a single multispectral or hyperspectral camera either virtually, in that the separate images recorded by the cameras 110, 111, 111a are processed as a single multispectral image, or as a single real multispectral camera which performs the functions of the different cameras 110, 111, 111a.

The respective fields of view 184 of the cameras 110, 111, and if present 111a, are preferably aligned or even coinciding and coaxial. It is preferred that the cameras 110, 111 provide the identical field of view 184 with the identical perspective and focal length. This results in identical representations of the object 106 in the images 112, 114 generated by the different cameras 110, 111. Both cameras 110, 111 may use the same objective 174.

If a match of the perspectives and field of view cannot be generated optically, it may be generated by image processing by applying a matching or registering routine to the digital input images 130, as is explained further below.

It is preferred that the cameras 110, 111, and, if present, 111a are operated synchronously. Specifically, the exposure times may be synchronized. Thus, the medical observation device 100 may be configured to generate the digital input images 130 at the same time.

Preferably, the gain of the at least two cameras 110, 111, 111a is synchronized, i.e. adjusted in the at least two cameras 110, 111, 111a at the same time. Moreover, the ratio of the gain applied in camera 110 to the gain applied in camera 111 and, if present, in camera 111a may be constant, even if the gain is changed. The gamma correction and color adjustment or white balance may be switched off or kept constant.

For separating the spectrum arriving at the digital reflectance input image 114 from the spectrum arriving at the at least one digital fluorescence-light input image 112, i.e. for separating the reflectance spectrum from the fluorescence spectrum, an optical color-separation assembly 176 may be provided. The color-separation assembly 176 may comprise optical elements such as a beam splitter 192, which may be dichroic. The color separation assembly 176 may further or alternatively comprise an optical observation filter set 188 and/or an optical fluorescence filter set 190.

The fluorescence filter set 190 is preferably configured to transmit light in the fluorescence spectrum or spectra of the one or more fluorophores 116, 118 and to block light outside the fluorescence spectrum or spectra.

The fluorescence filter set 190 may comprise one or more optical band-pass filters comprising one or more passbands. Each passband should overlap the fluorescence emission spectrum of a respective fluorophore 116, 118 of which the fluorescence is to be recorded. As the fluorescence-light filter set 190 is in the light path between the beam splitter 192 and the fluorescence-light color camera 111, only the wavelengths in the passbands of the fluorescence-light filter set 190 are transmitted to the fluorescence-light color camera 111.

If two fluorescence-light cameras 111, 111a are used to capture different fluorescence emission spectra, the fluorescence filter set 190 may comprise a different optical band-pass filter in front of each of the fluorescence-light color cameras 111, 111a. The passband of one band-pass filter may be contained in the fluorescence-emission spectrum of one fluorophore 116, whereas the passband of the other band-pass filter may be contained in the fluorescence-emission spectrum of another fluorophore 116, 118 in the object 106.

The observation filter set 188 is preferably configured to block light in the fluorescence spectrum or spectra of the one or more fluorophores 116, 118. The observation filter set 188 may also be configured to block light in the fluorescence-excitation spectrum.

The observation filter set 188 is preferably configured as a band-stop filter, of which the stopbands correspond to or at least contain the passbands of the fluorescence-light filter set 190. The observation filter set 188 is located in the light path between the beam splitter 192 and the white-light camera 110. Thus, white-light camera 110 records only wavelengths that are outside the stopbands of the observation filter set 188 and therefore also outside of the passbands of the fluorescence-light filter set 190.

Any one of the observation filter set 188 and the fluorescence filter set 190 may be a tunable filter.

If the beam splitter 192 is a dichroic beam splitter, at least one of the filter sets 188, 190 may be omitted as the optical spectral filtering in this case is already integrated in the dichroic beam splitter. The above description of the passbands and stopbands then should apply mutatis mutandis to the dichroic beam splitter 192.

The medical observation device 100 may further comprise an illumination assembly 178, which is configured to illuminate the object 106 preferably through the objective 174 through which the imaging system 102 records the at least one digital image 112, 114.

The illumination assembly 178 may be configured to selectively generate white-light, i.e. light that is evenly distributed across the entire visible spectrum, and fluorescence-excitation light, which contains light only in wavelengths that stimulate fluorescence of the at least one fluorophore 116, 118. The illumination light generated by the illumination assembly 178 may be fed into the objective 174 using an illumination beam splitter 180.

The illumination assembly 178 may be configured to generate illumination light simultaneously in one or a plurality of discrete, in particular narrow-band, wavelength bands. These wavelength bands may comprise any or any combination of the following wavelength bands.

One such discrete wavelength band may be entirely located in the fluorescence-excitation spectrum of a fluorophore 116. Another such wavelength band may be entirely located in the fluorescence-emission spectrum of another fluorophore 118. Another such wavelength band may be limited to wavelengths larger than 700 nm and be entirely located in the NIR range.

The simultaneous illumination of the object with any of the discrete wavelength bands as described above may be accomplished by a light source 199, e.g. a tunable light source such as a light source comprising a plurality of LEDs in different colors, in particular in different primary colors, which is configured to generate light in these wavelength bands simultaneously. Alternatively or additionally, the wavelength bands may be generated by using an illumination filter 179 having multiple passbands, wherein the passbands preferably correspond to the above wavelength bands. If such an illumination filter 179 is used, the light source 199 may generate white-light, which is then filtered by the illumination filter 179 so that only the light in the passbands illuminates the object 106.

The illumination filter 179 may be provided depending on the at least one fluorophore, of which fluorescence is to be triggered, and its specific excitation spectrum. For example, if 5-ALA is used as a fluorophore, the illumination filter may have a transmission of 90 % to 98 % up to wavelengths of 425 nm, a transmission between 0.5 % and 0.7 % in wavelengths between 450 nm and 460 nm, a transmission of not more than 0.1 % between 460 nm and 535 nm and of practically zero for wavelengths above 535 nm. The illumination filter 179 may be configured for pass-through of NIR light. For example, the illumination filter 179 may comprise a passband in the NIR. The illumination filter 179 may further comprise a passband, which is preferably entirely located in the fluorescence-excitation spectrum of another fluorophore.

The medical observation device 100 may be adjusted to a different fluorophore or set of fluorophores by re-configuring the color-separation assembly 176, e.g. by exchanging its optical elements, such as the filters set 190 and/or 188, or the dichroic beam splitter 192.

Using the set-up as described above, the medical observation device 100 is configured to record the fluorescence emission signal, i.e. the light emitted from the fluorescing fluorophore 116, 118 in two digital color input images 130, e.g. the digital fluorescence-light image 112 and the digital white-light color image 114. Both images 112, 114 then contain a part of the fluorescence emission signal.

Although the camera 110 is primarily used to record the anatomical background, i.e. a white-light reflectance image of the object 106, it may also be used to record part of the fluorescence emission of the at least one fluorophore 116, 118 if parts of the fluorescence emission spectrum leak into the passbands of the camera 110.

The input image data 120 are processed by a data processing device 170. The data processing device 170 may be an integral part of the medical observation device 100. In one example, the data processing device may be a processor, which is embedded in the medical observation device and also used as a controller for controlling the hardware of the medical observation device 100, such as the brightness and/or spectral emission of the light source 199 and/or any objective of the medical observation device 100 and/or any actuators of the medical observation device 100. In another example, the data processing device 170 is part of a general computer, which is connected to the medical observation device for unidirectional or bidirectional data transfer by wire or wirelessly.

The data processing device 170 may be a hardware module, such as a microprocessor, or a software module. The data processing device 170 may also be a combination of both a hardware module and a software module, for example by using software modules that are configured to be run on a specific processor, such as a vector processor, a floating point graphics processor, a parallel processor and/or on multiple processors. The data processing device 170 may be part of a general-purpose computer 186, such as a PC. In another embodiment, the data processing device 170 is an embedded system or embedded processor of the medical observation device 100.

The data processing device 170 is configured to access the input image data 120, e.g. in the form of the one or more digital input images 130, such as the digital white-light color input image 114 and the digital fluorescence-light image 112. The data processing device 170 may be configured to retrieve the digital input images 130 from a memory 194 and/or directly from the cameras 110, 111 and if present 111a. The memory 194 may be part of the data processing device 170 or reside elsewhere in the medical observation device 100.

The data processing device 170 is further configured to compute a digital output image 160 from the input image data 120.

The digital output image 160 is a color image, which is represented in a color space. The color space of the digital output image may be different from the color space of any digital color input image that is contained in the input image data 120. Preferably, however, the color space of the digital output image 160 is the same color space as that of any of the digital color input images 130.

A color space comprises at least three of such color channels. In the color space, each color channel is represented by a different color space coordinate. For conversion between different color spaces, color space transformations may be used. In a different color space, the same color is represented in different color space coordinates. Each pixel of the digital color input images 130 comprises a set of color space coordinates that together represent the color of the respective pixel. Each color band thus may be regarded as representing a color space axis and each color may be regarded as a point in color space, which is defined by the vector-i.e. the color space coordinates-pointing to this color. Thus, adding two colors corresponds to a vector addition. If one color has color space coordinates {x₁, y₁, z₁} and a second color has color space coordinate {x₂, y₂, z₂} then the sum of these two colors corresponds to the color {x₁+x₂, y₁+y₂, z₁+z₂}.

In one example, the digital color input images 130 or, more generally the input image data 120, may be recorded in RGB color space using the three primary colors or color bands-or color space coordinates-R, G, B. Alternatively, the digital color input image 130 may be recorded in different color spaces, respectively, and/or represent multispectral or hyperspectral color input images. The digital input images 130 of a set of digital input images, such as the digital white-light color input image 114 and the digital fluorescence color input image 112, need not be recorded in the same color space, although this is preferred.

In RGB color space, each color is represented by a triple of three color space coordinates in the form of integer numbers, wherein each integer number indicates the intensity of one of the primary colors R, G, B. For example, the most intense red is indicated by the triple [255 ,0, 0]. The most intense green color is indicated by [0, 255, 0], and the most intense blue by [0, 0, 255]. Thus, RGB color space is a three-dimensional space, CMYK color space would be a four-dimensional space. A color can be considered as a point in color space to which a vector such as [0, 0, 255] points. A multispectral or hyperspectral color space having n color bands would correspondingly result in an n-dimensional color space, in which each color is represented by an n-tuple of color space coordinates.

The digital output image 160 may be displayed on a display 132, which is integral with the medical observation device 100. For example, the display 132 may be integrated in an ocular or eyepiece 104 of the medical observation device 100.

The digital output image 160 is preferably generated in real-time, i.e. a digital output image 160 is generated from a set of digital color input images 130 before the next set is generated by the at least two cameras 110, 111, 111a.

The medical observation device 100 may comprise a direct optical path 134 from the object 106 through the objective 174 to the eyepiece 104. In such a case, the display may be a translucent display 132 located in the direct optical path 134 or the display may be projected into the direct optical path 134. A beam splitter 136 may be provided to split the light between the optical eyepiece 104 and the digital imaging system 102. In one embodiment, up to 80 % of the light may be directed to the eyepiece 104.

Alternatively, the medical observation device 100 may not have a direct optical path 134 but only display images from the integral display 132. As a further alternative, the medical observation device may not have any display at all.

The medical observation device 100 may comprise an output interface 172 to which one or more (external) displays 182 may be connected. For this, the output interface 172 may comprise standardized connectors and data transmission protocols, such as USB, HDMI, DVI, DisplayPort, Bluetooth and/or others. An external display may be a monitor, 3D goggles, oculars and the like. Any combination of external displays may be connected to output interface 172.

The computer 186 and/or the data processing device 170 is connected to the digital imaging system 102 using one or more data transmission lines 196. A data transmission line may be wired or wireless, or partly wired and partly wireless. The computer 186 and/or the data processing device 170 may not be bodily integrated in the medical observation device 100 but be physically located remote from the digital imaging system 102. For this, the digital imaging system 102 and the computer 186 and/or the data processing device 170 may be connected to a network, such as a LAN, a WLAN or a WAN, to which also at least one display 182 is connected.

According to a modification, the medical observation device 100 may be stereoscopic but comprise only two cameras, one for each stereoscopic channel. In one stereoscopic channel, the fluorescence-light color camera 111 is used and configured to selectively record white-light reflectance, whereas in the other stereoscopic channel, the white-light color camera 110 is used. Such an arrangement provides a stereoscopic white-light color input image if no fluorescence is used and a monoscopic white-light color input image and a monoscopic fluorescence-light color input image if fluorescence is used. The description above and below applies equally to this configuration.

The data processing device 170 may comprise a routine 140 for obtaining a set of signal descriptors that are representative of the (part of the) fluorescence emission signal contained in a digital color input image 130, 112, 114. In a very simple variant, the routine 140 simply extracts the set of color space coordinates of an input pixel of the digital input image as the set of signal descriptors. This variant is useful if no other signals than the fluorescence emission signal are contained in the respective digital input image, or if the other signals are negligible. In another case, the routine 140 may comprise spectral unmixing, which is then applied to a digital input image or an input pixel thereof, to obtain the set of signal descriptors. If spectral unmixing is used, the signal descriptors may be coefficients of endmembers.

In one embodiment, the routine 140 will obtain a separate set of signal descriptors for any digital input image 130 that is input to the routine 140. For example, if the routine 140 is presented with two digital input images 130, e.g. images 112, 114, it will obtain two separate sets of signal descriptors for each of the two digital input images.

In a variant, the routine 140 may obtain a single set of signal descriptors for any number of digital input images 130 input to routine 140.

The data processing device 170 may comprise a mapping routine 144, which is configured to assign a value to the set of signal descriptors obtained by the mapping routine 144. This value is then assigned to an output pixel of the digital output image, which output pixel is a corresponding pixel to the two input pixels from which the set of signal descriptors was computed. In one embodiment, the value may be a color, such as a pseudocolor or a false color. In another embodiment, the value may be contained in metadata that are assigned to the output pixel. As is explained further below, the value may in one preferred embodiment be representative of the chemical environment of the location of the object, which location is mapped to the two corresponding input pixels.

In case of a pseudocolor, a different color is assigned to each value. In case of a false color, a different intensity of the same hue is assigned to each value. Here, the expression color is assumed to refer to any (combination of) color appearance values.

The mapping routine 144 may use a linear transformation of the signal descriptors and e.g. use a color conversion matrix 146

Any of the routines 140 to 146 may be a software routine, a routine implemented in hardware, or a routine in which software and hardware components are combined. Any of the routines 140 to 146 may be stored in a memory 194 of the data processing device 170 or the medical observation device 100.

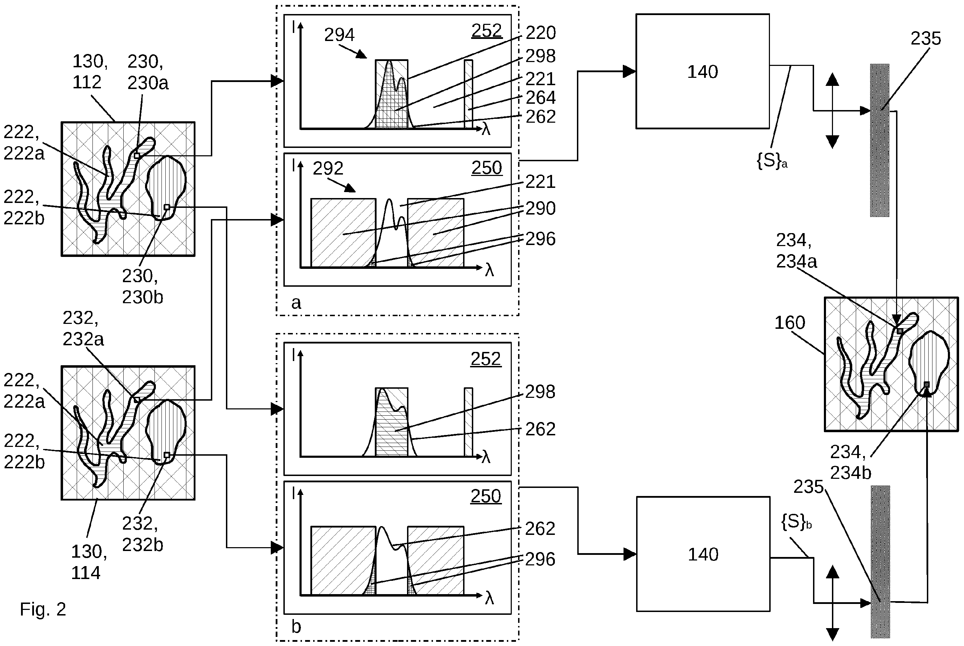

Fig. 2 shows how the digital output image 160 is generated from two digital color input images 130. A first digital color input image 130 may be recorded by camera 110 and thus contain the wavelengths that are used to represent the digital reflectance input image 114. A second digital color input image 130 may be recorded by one of the cameras 111, 111a and thus contain the wavelengths in which the at least one fluorophore 116, 118 fluoresces, such as the digital fluorescence input image 112. Of course, any two cameras of a medical observation device 100 may be used for recording the two digital color input images 130.

Each of the first and second digital color input images 130, 112, 114 contains a fluorescence emission signal 222, i.e. a component that is representative of the fluorescence emission of the at least one fluorophore 116, 118. In one particular embodiment, the object 106 contains just one fluorophore 116 such as 5-ALA/PplX. In this case, the fluorescence emission signal 222 contained in both digital color input images 130, 112, 114 corresponds to the fluorescence emitted by 5-ALA/PplX.

The data processing device 170 is preferably configured to identify and/or extract the fluorescence emission signal 222 in any of the first and second digital input images 130, 112, 114. This may be useful, if other signals such as a reflectance signal representative of light reflected off the object 106 is contained in any of the first and second digital input images 130, 112, 114 in addition to the fluorescence emission signal 222. The fluorescence emission signal 222 may e.g. be extracted from any of the first and/or second digital input image 130, 112, 114 by spectral unmixing. The fluorescence emission spectrum of the at least one fluorophore may be used as an endmember of the spectral unmixing.

The first digital color input image 114 is recorded in a first spectrum 250 and the second digital color input image 112 is recorded in a second spectrum 252 that is different from the first spectrum 250. In Fig. 2 the first and second spectra 250, 252 are indicated qualitatively by showing their normalized intensity I over wavelength λ. Preferably, the first and second spectra 250, 252 are non-overlapping and/or complementary. At wavelengths, where there is a stopband 221 in one of the two spectra 250, 252, there is a passband 220, 290 in the other of the two spectra 252, 250. In particular, both spectra 250, 252 may complement each other to represent the entire range of visible wavelengths. The visible wavelengths comprise light having wavelengths longer than about 380 nm and shorter than about 750 nm.

The first spectrum 250 comprises at least one passband 290 and at least one stopband 221. As shown, the first spectrum 250 may comprise two passbands 290 that are separated by a single stopband 221.

The second spectrum 252 comprises at least one passband 220. The passband 220 may comprise, be limited to or consist of the spectrum 262 of the fluorescence emission signal 222, i.e. the fluorescence emission spectrum of the at least one fluorophore 116, 118.

The second spectrum 252 may comprise a NIR passband 264 that comprises, consists of or is limited to wavelengths in the NIR range of light. The NIR range of light extends from about 750 nm to about 1400 nm or to about 2500 nm.

The passbands 220, 264 of the second spectrum 252 may be narrower than the at least one passband of the first spectrum 250. This allows rendering the reflectance spectrum of the object 106 more accurately and recording a more faithful white-light image in other operating modes of the medical observation device 100.

The fluorescence emission spectrum 262 preferably overlaps at least one passband 220 of the first spectrum 290 and at least one passband 220 of the second spectrum 252. Preferably, more than one passband 290 of the first spectrum 250 overlaps the fluorescence emission spectrum 262. In one instance, the NIR passband 264 may also overlap the fluorescence emission spectrum 262.

Thus, a first part 296 of the fluorescence emission spectrum 262 is contained in the first spectrum 250 and a second part 298 of the fluorescence emission spectrum 262 is contained in the second spectrum 252.

By recording the fluorescence emission spectrum 262 in two different spectra 250, 252 of two digital color input images 130, 112, 114, the spectral resolution of the fluorescence emission signal 222 is increased. This allows for a more accurate analysis of the fluorescence emission spectrum 262 compared to a single digital color input image or to two or more digital color input images that are recorded in identical spectra.

The fluorescence emission spectrum 262 of the at least one fluorophore 116, 118 may depend on the environment. In particular, the fluorescence emission spectrum 262 may depend on the presence of certain ions and molecules in the environment of the at least one fluorophore 116, 118. For example, the fluorescence spectrum of the at least one fluorophore 116, 118 in an alkaline environment may be different from the fluorescence emission spectrum in an acid environment. The chemical environment of tumorous biological tissue in turn may depend on the state of the biological tissue 107 in the object 106. According to one aspect, the fluorescence emission spectrum of the at least one fluorophore may depend on the characteristics of a tumor, in particular its grading, as different tumor types may create different chemical environments. Thus, the fluorescence emission spectrum 262 may be representative of the characteristics of the tumor for specific fluorophores, such as 5-ALA/PplX.

The environment-dependent change in the fluorescence emission spectrum 262 may involve at least one of a shift in a peak emission wavelength, wherein, if more than one peak is present, any peak may be affected; the emergence of secondary or tertiary (or more) peaks in the fluorescence emission spectrum; the width of the fluorescence spectrum 262, its symmetry, the intensity in the off-peak wavelengths and/or other geometrical characteristics of the fluorescence emission spectrum 262. Thus, the fluorescence spectrum 262 of the fluorescence emission signal 222 may vary at different locations of the object 106 depending on the chemical environment at this location.

As each location of the object 106 within the field of view 184 is mapped to a first input pixel 230 of the first digital color input image 112 and to a corresponding second input pixel 232 in the second digital color input image 232, the fluorescence emission spectrum 262 may vary depending on the location of the pair of corresponding input pixels 230, 232 in the first and second digital color input image 112, 114.

In Fig. 2, the fluorescence emission signal 222 in one area 222a of a digital input image 130, 112, 114 and thus of the object 106 differs from the fluorescence emission signal 222 in another area 222b. For example, area 222a may represent an area of the object 106 where the chemical environment has different characteristics than in the area of the object 106 that is mapped to area 222b. For example, a chemical parameter such as the pH level may be different in area 222a and in area 222b. For example, the area 222a may comprise tumorous tissue having a higher tumor grade than the tumorous tissue in area 222b. The higher tumor grade may correspond to a higher pH level.

Box a in Fig. 2 shows a sample fluorescence spectrum 262 at a first input pixel 230a and a corresponding second input pixel 232a that are located both in the area 222a. Box b shows a sample fluorescence spectrum 262 of a first input pixel 230b and a corresponding second input pixel 232b in the area 222b.

For each pair of corresponding input pixels 230, 232, such as the pair 230a and 232a and/or the pair 230b and 232b, the data processing device 170 obtains at least one set {S} of signal descriptors S that is representative of the fluorescence emission signal 222 recorded in that pair. According to one aspect, a different set of signal descriptors S may be obtained for each part 296, 298 of the fluorescence emission signal 222 at a pixel 230, 232 respectively. According to another aspect, a single set of signal descriptors S may be obtained for each pair of corresponding pixels 230, 232, respectively.

Different shapes of the fluorescence emission spectrum 262 lead to different sets of signal descriptors {S}. The signal descriptors S may be scalar values such as coefficients of polynomials or any other orthogonal set of functions, color space coordinates or coefficients of endmembers in spectral unmixing. The set {S} of signal descriptors may be a vector or a matrix.

For example, in area 222a, a set {S}ₐ of signal descriptors may be obtained that is representative of the fluorescence emission spectrum 262 in this area. In area 222b, a different set {S}_{b} of signal descriptors may be obtained that is representative of the fluorescence emission spectrum in this area. The set of signal descriptors may thus be used to differentiate between the different environments of the at least one fluorescing fluorophore 116, 118, e.g. between different tumor types and/or grades.

Each set of signal descriptors may be assigned a different value. The value is assigned to an output pixel in the digital output image 160 either as a color or in the metadata of this pixel. The mapping of a set {S} to a value preferably is bijective. The color of an output pixel 234 of the digital output image 160 thus depends on the set of signal descriptors derived from the pair of corresponding input pixels 230, 232. The output pixel 234 is a corresponding pixel to the pair 230, 232.

For example, an output pixel 234a corresponding to input pixels 230a, 232a in the area 222a may be assigned a different hue than the output pixel 234b corresponding to input pixels 230b, 232b in area 222b. The intensity of the hue at an output pixel 234 may correspond to the intensity of the fluorescence emission signal 222 at the corresponding pair of input pixels 230, 232.

In the example given in Fig. 2, the fluorescence emission signal 222 in area 222a is assigned a reddish hue, whereas the fluorescence emission signal 222 in area 222b is assigned a bluish hue. Correspondingly, output pixel 234a is red and output pixel 234b is blue.

The set {S} may be mapped to any color appearance parameter or combination of color appearance parameters.

The computation of the set {S} of signal descriptors may be done in the medical observation device 100 of Fig. 1 by the routine 140. The assignment of value, in particular of a color 235 to an output pixel 234 depending on the set {S} of signal descriptors S may be done using the mapping routine 144.

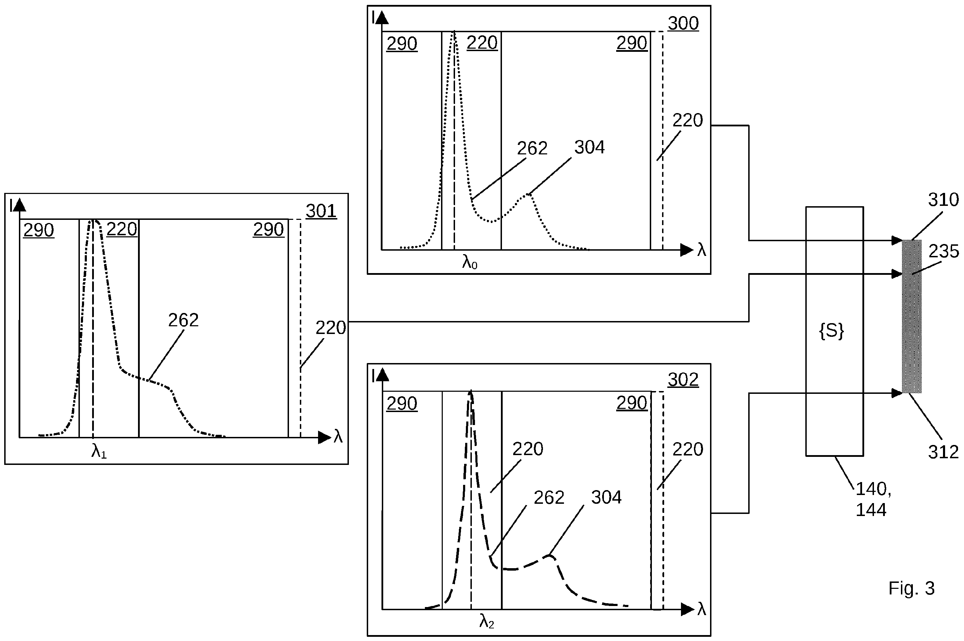

Fig. 3 indicates how the fluorescence emission spectrum of at least one fluorophore 116, 118 may change depending on the chemical environment in which the at least one fluorophore is located. The chemical environment may be classified using a chemical parameter, which represents for example, the concentration of particular molecules or ions.

In particular, Fig. 3 shows a quantitative example of the fluorescence emission spectrum case of 5-ALA/PplX. Details on how the fluorescence emission spectrum of 5-ALA/PpIX changes depending on the pH values are e.g. given in Montcel, B., et al. (2013) "Two-peaked 5-ALA-induced PpIX fluorescence emission spectrum distinguishes glioblastomas from low grade gliomas an infiltrative component of glioblastomas", Biomedical Optics Express 4(4), p. 550-558.

The spectrum 300 presents a sample fluorescence emission spectrum 262 of the fluorescence emission signal of 5-ALA/PpIX in a first chemical environment, e.g. a highly alkaline environment. The fluorescence emission spectrum 262 is recorded in the passbands 290, 220 of the first and second spectrum 250, 252 as explained with reference to Fig. 2. In a highly alkaline environment, an emission peak of 5-ALA is located at a wavelength of about λ₀=620 nm. At lower frequencies, a secondary peak 304 may be identified.

In a highly acid environment, the fluorescence emission spectrum 262 may take the form as shown at 302. The peak emission frequency λ₂ may have shifted to 634 nm and the sideband at lower frequency may contain more energy, leading to a less marked secondary peak 304.

In environments that are less alkaline than the environments which result in the spectrum 300, or less acid than the environments which result in the spectrum 302, the fluorophore may exhibit a spectrum 301. The peak emission frequency λ₁ may be located between λ₀ and λ₂ or the fluorescence emission spectrum may exhibit two peaks at λ₀=620 nm and λ₂=634 nm. The spectrum 301 may be a superposition of the two spectra 300 and 302, the proportions of the spectrum 300 and 302 being indicative of the pH level of the environment of the fluorescing fluorophore.

The data processing device 170 will obtain a set {S} of signal descriptors S which is different for the different spectra 300, 301, 302. For obtaining the set {S}, the data processing device 170 may use routine 140. The routine 140 may use spectral unmixing to compute the set {S}, using the spectra 300 and 302 as endmembers.

Each of the different sets {S} may be mapped to a different value, in particular color 235, using the mapping routine 144 as explained above. The value is selected from a range of values that extends from a first value to a second value. If the value corresponds to a color, the color 235 is selected from a range of colors that extend from a first color 310 to a second color 312.

Of course, any other cause that changes the fluorescence emission spectrum of the fluorophore may be reflected in the set {S}. For example, instead of using pH-dependent spectra 300 as endmembers, spectra (not shown) may be chosen that are representative of another chemical parameter, e.g. the concentration of any biomarkers. In particular, the endmembers may be chosen such that they reflect a chemical environment that is typical for certain tissue types or states that need to be indicated in the digital output image 160.

According to one aspect, the passbands 220, 290 may be used as endmembers.

In the example shown in Fig. 3, the signal descriptors {S} representing the spectrum 300 of the fluorophore in the highly acid, preferably most acid, environment is assigned the first color 310. The signal descriptors representing the spectrum 302 of the fluorophore in the highly alkaline, preferably most alkaline, environment is assigned the second color 312. The signal descriptors representative of the spectrum 301-i.e. of the fluorophore in an environment of which the pH value is between the highly acid and the highly alkaline level-will be assigned a color of which at least one color appearance parameter is located between the at least one respective color appearance parameter of the colors 310 and 312.

The value for a spectrum 301 may be obtained by linearly mapping the set {S} of signal descriptors onto the predetermined range of values. In case of a color, the value may directly correspond to a color in the predetermined color range; by alpha blending the first and second color 310, 312 using a transparency value for one of the first and second color that depends on the set {S}; by weighted vector addition of the first and second color 310, 312; or by providing a look-up table or an analytical function that maps a set {S} to a color 235.

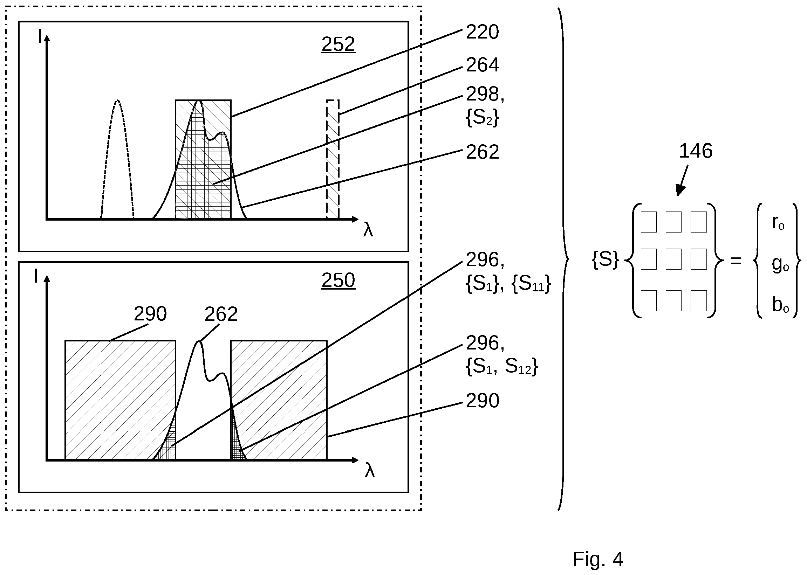

As shown in Fig. 4, a set {S} containing any number of signal descriptors S may be obtained from the two digital input images 112, 114, or their spectra 250, 252, respectively.

For example, a set {S₂} of signal descriptors S₂ which is representative of the second part 298 of the fluorescence emission signal at a second input pixel 232 may simply contain the color space coordinates of the second input pixel 232. If the second input image 130, 112 is represented in RGB color space, the set {S₂} may thus correspond to {r₂, g₂, b₂}.

If spectral unmixing is used, the set {S₂} may correspond to the coefficients of the endmembers at the second input pixel 232. For example, the signal descriptor S₂ may correspond to the coefficient that represents the contribution of the fluorescence emission spectrum 262 of 5-ALA/PpIX in an alkaline or in an acid environment. In another example of spectral unmixing, the set {S₂} may contain two signal descriptors {S₂₁, S₂₂}, one signal descriptor S₂₁ corresponding to the coefficient that represents the contribution of the fluorescence emission spectrum 262 of 5-ALA/PpIX in an alkaline environment, the other signal descriptor S₂₂ corresponding to the coefficient that represents the contribution of the fluorescence emission spectrum 262 of 5-ALA/PpIX in an acid environment.

In again another embodiment, the passband 220 itself may be used as an endmember, resulting in a set {S₂}, which contains a single signal descriptor.

The same may apply to a first input pixel 230 in the first digital input image 130, 114.

Again, the set of color coordinates of the first input pixel 230 may be used as a set {S₁} of signal descriptors of the fluorescence emission signal at the first input pixel. In RGB color space, {S₁} thus may simply correspond to {r₁, g₁, b₁}. In this case, {S₁} is representative for both parts 296 of the fluorescence emission signal in the first and second passbands 290.

If spectral unmixing is used, the set {S₁} of signal descriptors may correspond to the coefficients that represent the respective contributions of the endmembers to the fluorescence emission signal at the first input pixel 232 in the passbands 290. The number of signal descriptors in the set {S₁} depends on the number of endmembers used in the spectral unmixing.

For example, the signal descriptor S₁ may correspond to the coefficient that represents the contribution of the fluorescence emission spectrum 262 of 5-ALA/PpIX in an alkaline or in an acid environment. In another example of spectral unmixing, the set {S₁} may contain two signal descriptors, e.g. {S₁₁, S₁₂}, one signal descriptor S₁₁ corresponding to the coefficient that represents the contribution of the fluorescence emission spectrum 262 of 5-ALA/PpIX in an alkaline environment, the other signal descriptor S₁₂ corresponding to the coefficient that represents the contribution of the fluorescence emission spectrum 262 of 5-ALA/PpIX in an acid environment.

In again another embodiment, each of the passbands 220 itself may be used as an endmember, resulting in a set {S₁}, which contains two signal descriptors, one signal descriptor representing the content of spectrum 250 in the low-frequency passband 290 and the other signal descriptor representing the content of spectrum 250 in the high-frequency passband 290.

The fluorescence emission signal recorded in the pair of corresponding pixels 230, 232 may be represented by the union set {S} = {S₁, S₂} of the signal descriptors obtained from the two digital input color images 112, 114, e.g. {r₁, r₂, g₁, g₂, b₁, b₂}, {S₁₁, S₁₂, S₂}, or {S₁₁, S₁₂, S₂₁, S₂₂}.

Independent of how the set {S} of signal descriptors was obtained-e.g. by using the color space coordinates or by applying spectral unmixing-and how many and what kind of signal descriptors it contains, the set {S} may be mapped to a value, in particular a color 235, which is then assigned to the output pixel 234. According to one aspect, the assignment of a color can be done using the color conversion matrix 146 which is multiplied with the set {S}-treated as a vector-to obtain the color space coordinates of the output pixel 234. If the digital output image 160 is for example represented in RGB color space, the color space coordinates of the output pixel 234 obtained by applying the color conversion matrix 146 may be {rₒ, gₒ, bₒ}.

The color conversion matrix 146 may have a first dimension which corresponds to the number of signal descriptors in the set {S} and a second dimension which corresponds to the number of color space coordinates of the output pixel 234. If the set {S} contains the color space coordinates of the first and second input pixels 230, 232, the first dimension may be six. If the set {S} corresponds to {S₁₁, S₁₂, S₂}, the first dimension is three, if the set {S} corresponds to {S₁₁, S₁₂, S₂₁, S₂₂}, the first dimension is four. If the output color image is a RGB image, the second dimension is three.

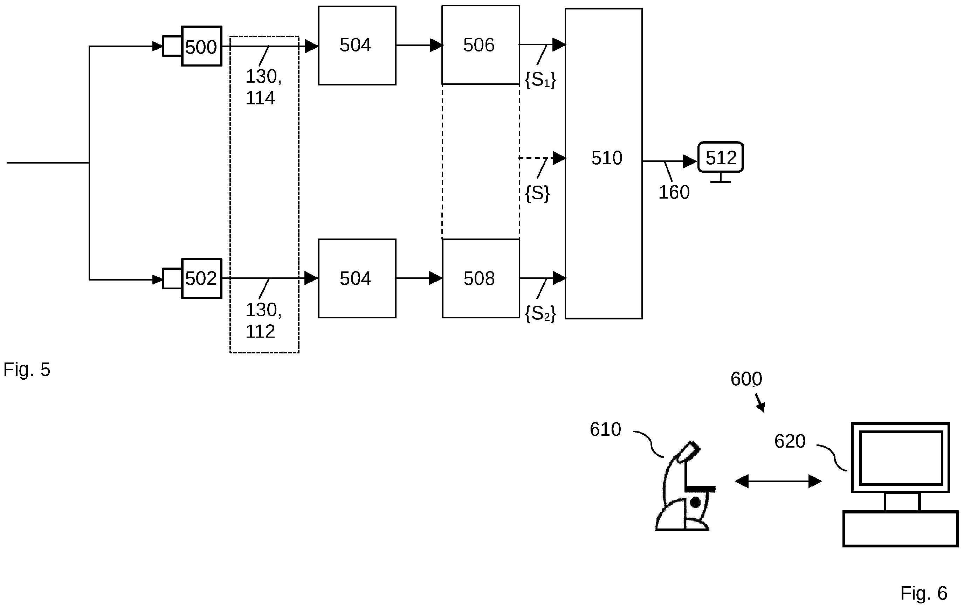

Fig. 5 presents an overview of a method for obtaining a digital output image 160.

First, at step 500, a first digital color input image 130 is obtained, e.g. the digital reflectance image 114, which contains a part of the fluorescence emission signal 222.

At step 502, a second digital color input image 130 is obtained, e.g. the digital fluorescence input image 112, which contains another part of the fluorescence emission signal 222. Preferably, the two parts of the fluorescence emission signal 222 as contained in the first and second digital color input image 130 are complementary. Together, they may represent the entire or at least a major part of the spectrum 262 of the fluorescence emission signal 222 in the visible light range and optionally also the NIR range.

The first and/or second digital input image 130 may be obtained from a storage device, such as a computer memory or a hard disk, or directly from a camera 108.

At optional step 504, the fluorescence emission signal 222 may be extracted from the first and/or second digital color input image 130, 112, 114. This may be necessary to separate the fluorescence emission signal 222 from other signals that may be present in a digital color input image, such as parts of the fluorescence excitation spectrum.

At step 506, a set {S₁} of signal descriptors is obtained from the first digital input image 130, 114.

At step 508, a set {S₂} of signal descriptors is obtained from the second digital input image 130, 112. The sets {S₁}, {S₂} are representative of the respective parts of the fluorescence emission signal 222 or its spectrum 262 in the first and second digital input image. In one variant, steps 506 and 508 may be combined in a single step, as indicated by the dotted lines, which results directly in a single set {S} of signal descriptors S for both the first and the second digital color input image 112, 114.

At step 510, a value or a color of an output pixel 234 of the digital output image 160 is assigned to the set of signal descriptors, either the union set {S₁, S₂} or the set {S}.

The digital output image 160 may then be displayed at step 512, e.g. using a display 182.

Some embodiments relate to a microscope comprising a system as described in connection with one or more of the Figs. 1 to 5. Alternatively, a microscope may be part of or connected to a system as described in connection with one or more of the Figs. 1 to 5.

Fig. 6 shows a schematic illustration of a system 600 configured to perform a method described herein. The system 600 comprises a microscope 610 and a computer system 620. The microscope 610 is configured to take images and is connected to the computer system 620. The computer system 620 is configured to execute at least a part of a method described herein. The computer system 620 may be configured to execute a machine learning algorithm. The computer system 620 and microscope 610 may be separate entities but can also be integrated together in one common housing. The computer system 620 may be part of a central processing system of the microscope 610 and/or the computer system 620 may be part of a subcomponent of the microscope 610, such as a sensor, an actor, a camera or an illumination unit, etc. of the microscope 610.

The computer system 620 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 620 may comprise any circuit or combination of circuits. In one embodiment, the computer system 620 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 620 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 620 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 620 may also include a display device, one or more speakers, and a keyboard and/or controller, which can include a mouse, trackball, touch screen, voice-recognition device, or any other device that permits a system user to input information into and receive information from the computer system 620.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### Reference Numerals

- 100: medical observation device
- 101L: stereoscopic subassembly for the left channel
- 101R: stereoscopic subassembly for the right channel
- 102: digital imaging system
- 104: eyepiece
- 106: object
- 107: biological tissue
- 108: digital camera
- 110: digital reflectance camera
- 111: digital fluorescence camera
- 111a: second digital fluorescence camera
- 112: digital fluorescence input image
- 114: digital reflectance input image
- 116: fluorophore, e.g. fluorophore naturally contained in object
- 118: fluorophore, e.g. fluorophore artificially added to object
- 120: input image data
- 130: digital input image
- 132: internal display
- 134: direct optical path
- 136: beam splitter
- 140: separation or extraction routine
- 144: mapping routine
- 146: color conversion matrix
- 160: digital output image
- 170: data processing device
- 172: output interface
- 174: objective
- 176: color-separation assembly
- 178: illumination assembly
- 179: illumination filter
- 180: illumination beam splitter
- 182: display
- 184: field of view
- 186: computer
- 188: observation filter set
- 190: fluorescence filter set
- 192: dichroic beam splitter
- 194: memory
- 196: data transmission line
- 199: light source
- 220: fluorescence passband
- 221: stopband
- 222: fluorescence emission signal
- 230: first color input pixel
- 232: second color input pixel
- 234: color output pixel
- 235: color
- 250: first spectrum (at first color input pixel) of first digital input color image
- 252: second spectrum (at second color input pixel) of second digital input color image
- 262: spectrum of fluorescence emission signal, fluorescence emission spectrum
- 264: NIR passband
- 290: spectral band of first spectrum
- 292: first spectrum
- 294: second spectrum
- 296: first part of fluorescence emission signal/spectrum
- 298: second part of fluorescence emission signal/spectrum
- 300: quantitative spectrum of 5-ALA/PpIX in acidic environment
- 301: quantitative spectrum of 5-ALA/PpIX in intermediate acid to alkaline environment
- 302: quantitative spectrum of 5-ALA/PpIX in alkaline environment
- 304: secondary peak
- 310: first color
- 312: second color
- 500, 502: obtaining digital input image
- 504: extracting fluorescence emission signal from digital input image
- 506: obtaining set of signal descriptors from first digital input image
- 508: obtaining set of signal descriptors form second digital input image
- 510: step of assigning a color to the set of signal descriptors
- 512: step of displaying digital output image
- 600: system
- 610: microscope
- 620: computer system
- I: intensity
- λ: wavelength
- λ0, λ1, λ2: peak emission wavelengths
- S, S1, S2, S11, ...: signal descriptors
- r1, g1, b1: color space coordinates of first input pixel
- r2, g2, b2: color space coordinates of second input pixel
- ro, go, bo: color space coordinates of color output pixel
- {..}: set

## Claims

1. Data processing device (170) for a medical observation device (100), such as a microscope or endoscope, for observing an object (106) containing at least one fluorophore (116, 118) that emits fluorescence,
wherein the data processing device is configured:
- to obtain a first color input pixel (230) of a first digital color input image (130, 114) recorded in a first spectrum (292) and containing a first part (296) of a fluorescence emission signal (222), the fluorescence emission signal being representative of the fluorescence emitted by the at least one fluorophore;
- to obtain a second color input pixel (232) of a second digital color input image (130, 112) recorded in a second spectrum (294) and containing a second part (298) of the fluorescence emission signal, the second spectrum being different from the first spectrum;
- to obtain a first set ({S₁}) of signal descriptors (S) representative of the first part of the fluorescence emission signal;
- to obtain a second set ({S₂}) of signal descriptors representative of the second part of the fluorescence emission signal; and
- to assign a value (235) to an output pixel (234) in a digital output image (160) depending on the first set of signal descriptors and the second set of signal descriptors, the value being representative of a chemical environment of the at least one fluorescence-emitting fluorophore.

2. Data processing device according to claim 1,
wherein the value (235) is a color.

3. Data processing device according to claim 1 or 2,
wherein the value is representative of a chemical environment at a location of the object, in which location the at least one fluorescence-emitting fluorophore (116, 118) is located, the location being mapped to the first and second color input pixel (230, 232).

4. Data processing device (170) according to any one of claims 1 to 3,
wherein the first color input pixel (230) comprises first color space coordinates {r₁, g₁, b₁};
the second color input pixel (232) comprises second color space coordinates {r₂, g₂, b₂};
the first set ({S₁}) of signal descriptors (S₁) comprises the first color space coordinates; and
the second set ({S₂}) of signal descriptors (S₁) comprises the second color space coordinates.

5. Data processing device (170) according to any one of claims 1 to 4,
wherein the data processing device is configured
- to apply spectral unmixing to the first color input pixel (230) to obtain the first set of signal descriptors (S_{I}, S_{III}); and
- to apply spectral unmixing to the second color input pixel (232) to obtain the second set of signal descriptors (S_{II}).

6. Data processing device (170) according to claim 5,
wherein the first spectrum (292) comprises at least two discrete spectral bands (290) and each of the two spectral bands is an endmember of the spectral unmixing.

7. Data processing device (170) according to claim 6,
wherein the second spectrum (294) is located between the two spectral bands (290) of the first spectrum (292).

8. Data processing device (170) according to any one of claims 2 to 7,
wherein the data processing device (170) is configured
- to assign the color (235) to the color output pixel (234) by applying a mapping routine (144) to a union set ({S₁, S₂}) of the first set and the second set of signal descriptors (S₁, S₂).

9. Data processing device according to any one of claims 1 to 8,
wherein the mapping routine (144) is configured
- to map the first and second set ({S₁}, {S₂}) of signal descriptors (S₁, S₂) to a first value, if the first and second signal sets are representative of a fluorescence emission spectrum (262) of the at least one fluorophore in a first chemical environment, the first chemical environment being represented by a first figure of a chemical parameter;
- to map the first and second set to a second value, if the first and second sets are representative of a fluorescence emission spectrum (262) of the at least one fluorophore in a second chemical environment, the second chemical environment being represented by a second figure of the chemical parameter, the first figure being different from the second figure;
- to map the first and second set to a third value located between the first and the second value, if the first and second sets are representative of the fluorescence emission spectrum (262) of the at least one fluorophore in a third chemical environment, the third chemical environment being represented by a third figure of the chemical parameter, the third figure being located between the first and the second figure.

10. Medical observation device (100) for observing an object (106) containing at least one fluorescing fluorophore (116, 118), such as a microscope or endoscope,
the medical observation device comprising:
- the digital processing device (170) according to any one of claims 1 to 9;
- a first digital color camera (110) for recording the first digital color input image (114) of the object in the first spectrum (292); and
- a second digital color camera (111) for recording the second digital color input image (112) of the object in the second spectrum (294).

11. Medical observation device (100) according to claim 10,
wherein the medical observation device comprises:
- an observation filter set (188) located in front of the first digital color camera (110) and comprising an observation stopband (221);
- a fluorescence filter set (190) located in front of the second digital color camera (111) and comprising a fluorescence passband (220);
wherein the fluorescence passband is located in the observation stopband.

12. Computer-implemented method for a medical observation device (100),
wherein the computer-implemented method comprises the following steps:
- obtaining a first color input pixel (230) of a first digital color input image (130, 114) recorded in a first spectrum (292) and containing a first part (296) of a fluorescence emission signal (222), the fluorescence emission signal being representative of the fluorescence emitted by the at least one fluorophore;
- obtaining a second color input pixel (232) of a second digital color input image (130, 112) recorded in a second spectrum (294) and containing a second part (298) of the fluorescence emission signal, the second spectrum being different from the first spectrum;
- obtaining a first set ({S₁}) of signal descriptors (S) representative of the first part of the fluorescence emission signal;
- obtaining a second set ({S₂}) of signal descriptors representative of the second part of the fluorescence emission signal; and
- assigning a value (235) to an output pixel (234) in a digital output image (160) depending on the first set of signal descriptors and the second set of signal descriptors, the value being representative of a chemical environment of the at least one fluorescence-emitting fluorophore.

13. Method for using a medical observation device, for observing an object (106) containing at least one fluorescing fluorophore (116, 118), such as a microscope or endoscope,
the method comprising the steps of
- recording the first digital color input image (130, 114) in a first spectrum (292), the first digital color input image comprising a plurality of first color input pixels (230);
- recording a second digital color input image (130,112) in a second spectrum (294), the second digital color input image comprising a plurality of second color input pixels (232), the first spectrum being different from the second spectrum; and
- carrying out the computer-implemented method of claim 12.

14. Computer program product computer-readable medium comprising instructions, which, when the program is executed by a computer, cause the computer to carry out the method of claim 12.

15. Computer-readable medium comprising instructions, which, when the program is executed by a computer, cause the computer to carry out the method of claim 12.
